# EUROPEAN PATENT APPLICATION

(11) **EP 4 331 636 A1**
(43) Date of publication of application: **06.03.2024**
(21) Application number: 22795383.3
(22) Date of filing: 23.03.2022
(51) Int. Cl.: A61L 101/22, A61L 2/18

(54) **INDOOR DECONTAMINATION DEVICE**

(30) Priority: 26.04.2021 JP 2021073754
(71) Applicant: Airex Co., Ltd., Nagoya-shi Aichi 453-0015 (JP)
(72) Inventor: KAWASAKI, Koji, Nagoya-shi Aichi 453-0015 (JP); KAKUDA, Daisuke, Nagoya-shi Aichi 453-0015 (JP); KITAHORA, Kazuhiko, Nagoya-shi Aichi 453-0015 (JP); FUTAMURA, Haruka, Nagoya-shi Aichi 453-0015 (JP); OGATA, Yoshitaka, Nagoya-shi Aichi 453-0015 (JP)
(74) Representative: Kraus & Lederer PartGmbB
(86) International application number: PCT/JP2022/013558
(87) International publication number: WO 2022/230463

(57) **Abstract**

An indoor decontamination device which can realize a perfect decontamination effect by supplying a proper amount of a decontamination mist to each corner of a decontamination target room and improve an efficiency of a decontamination work by shortening a work time for an aeration or the like is provided.

A fine decontamination mist with a favorable decontamination efficiency is employed, and a decontamination device main-body and a mist discharging dispersion device are provided. The decontamination device main-body includes a drive means and a liquid supply means for a decontamination chemical solution. The mist discharging dispersion device includes a mist discharging means and a mist dispersion means. The mist discharging means converts the decontamination chemical solution to the decontamination mist and supplies it into an inside of a work room. The mist dispersion means causes an acoustic flow to be generated by subjecting a vibration board to an ultrasonic vibration, causes a pressing force by an acoustic radiation pressure to act on the decontamination mist supplied into the work room, and causes the decontamination mist to be dispersed into the work room.

## Description

### TECHNICAL FIELD

The present invention relates to an indoor decontamination device for decontaminating indoor wall surfaces and an indoor equipment by discharging a decontamination mist into rooms such as a sterile room, a clean room, a hospital room and the like and particularly relates to an indoor decontamination device including a mist discharging dispersion device and is movable as necessary.

### BACKGROUND ART

Conventionally, an indoor decontamination device which is installed in a room such as a sterile room, a clean room, a hospital room and the like and decontaminates wall surfaces in a decontamination target room and an equipment in the room by discharging a decontamination gas is known. The conventional decontamination device includes a decontamination-gas generating device in a main body. The decontamination gas generated from this decontamination-gas generating device is directly discharged into the room from a decontamination-gas discharge port of a main body and decontaminates an inside of the decontamination target room.

By the way, when a decontamination target room is to be decontaminated by the indoor decontamination device, it was necessary to discharge the decontamination gas from an appropriate place in the room so that the decontamination gas smoothly spreads to each corner of the decontamination target room. However, in the case of the conventional indoor decontamination device, a main body which incorporates the decontamination-gas generating device is large-sized, and a place for the installation in the decontamination target is limited in some cases. Thus, the decontamination gas could not be discharged from an appropriate place, and the decontamination in the room became insufficient or the decontamination took time, which were problems.

Moreover, when a decontamination target room having a complicated shape or an obstacle in the room is to be decontaminated, it was difficult to spread the decontamination gas to each corner only by discharging it from one spot. Thus, there was also a problem that a plurality of the indoor decontamination devices should be disposed in the decontamination target room. Thus, the applicant proposed an indoor decontamination device according to the Patent Literature 1 described below.

This indoor decontamination device is constituted by a parent machine installed in the decontamination target room and a plurality of children machines connected to that. The parent machine and the children machines are connected via a decontamination-gas supply pipe. Moreover, in this indoor decontamination device, since both the parent machine and the child machine include the decontamination-gas discharge port, the decontamination gas can be discharged from a plurality of spots in the decontamination target room.

On the other hand, a hydrogen peroxide has been widely employed in recent years for the decontamination. This hydrogen peroxide has a strong sterilization effect, is unexpensive, is widely available, and is finally decomposed into an oxygen and a water, which is effective as an environmentally friendly decontamination gas.

This decontamination effect by the hydrogen peroxide is enabled by a condensed film of a hydrogen peroxide solution condensed on a surface of a decontamination target part as described in the following Patent Literature 2. Therefore, in order to realize a perfect decontamination effect of the decontamination target room, it is only necessary to increase a supply amount of the hydrogen peroxide and to thicken or to increase a concentration of the condensed film of the hydrogen peroxide solution to be genereated.

### CITATION LIST

### PATENT LITERATURE

Patent Document 1: Japanese Patent Application Publication No. 2009-273645
Patent Document 2: Japanese Patent Publication No. 61-004543

### SUMMARY OF INVENTION

### TECHNICAL PROBLEM

By the way, when an excessive amount of the hydrogen peroxide is supplied to the decontamination target room, an excessive condensation occurs, and such a nonconformity occurs that wall surfaces of the decontamination target room and various devices and the like installed therein are corroded by a condensed film of the generated hydrogen peroxide solution with a high concentration. Moreover, after a decontamination by the hydrogen peroxide, an aeration for removing the hydrogen peroxide or the condensed film remaining inside the decontamination target room by a clean air is performed. However, when the excessive amount of the hydrogen peroxide solution was supplied, there was a problem that a longer time is needed for the aeration for removing the condensed film of a high-concentration hydrogen peroxide solution generated on the wall surfaces or the like of the decontamination target room.

Thus, the present invention has, in order to deal with the aforementioned problems, an object to provide an indoor decontamination device which can realize a perfect decontamination effect by employing the fine decontamination mist with a favorable decontamination efficiency and by supplying a proper amount of a decontamination mist to each corner of a decontamination target room and improve an efficiency of a decontamination work by shortening a work time for an aeration or the like.

### SOLUTION TO PROBLEM

In order to solve the aforementioned problems, after an extended research, the inventors considered that the aforementioned object can be achieved by employing a mist discharging dispersion device using an ultrasonic vibration for an indoor decontamination device and by making the indoor decontamination device movable as necessary, and the present invention was accomplished.

That is, an indoor decontamination device according to the present invention includes, according to a description in claim 1,
an indoor decontamination device (100, 200) which is disposed in a work room (CR) and decontaminates an inside of an entire room, having:
a decontamination device main-body (10, 40) and a mist discharging dispersion device (30, 60) connected to the decontamination device main-body directly or via a connecting device (20, 50), characterized in that
the decontamination device main-body includes a drive means and a supply means which supplies a decontamination chemical solution to the mist discharging dispersion device;
the mist discharging dispersion device includes a mist discharging means (32, 62) and a mist dispersion means (33, 63);
the mist discharging means converts the decontamination chemical solution to a decontamination mist and supplies it into the inside of the work room; and
the mist dispersion means includes a board (34, 64) on which a plurality of transmitters (33a, 63a) are disposed, causes an acoustic wave by an ultrasonic wave to be generated in a vertical direction from a board surface of the board by subjecting the plurality of transmitters to an ultrasonic vibration, causes a pressing force by an acoustic radiation pressure to act on the decontamination mist supplied into the work room and disperses the decontamination mist in the work room.

Moreover, the present invention is, according to the description in claim 2, the indoor decontamination device described in claim 1, characterized in that
the plurality of transmitters are disposed on a board surface of the board in a unified wave-transmission direction and are operated in a same phase, whereby
ultrasonic waves in a front-surface direction of the plurality of transmitters reinforce each other, the ultrasonic waves in a lateral direction of the plurality of transmitters cancel each other, and an acoustic flow by the ultrasonic waves with a strong directivity in a vertical direction is generated from the board surface of the board.

Moreover, the present invention is, according to the description in claim 3, the indoor decontamination device described in claim 1 or 2, characterized in that
the mist discharging dispersion device includes a unified unit (31, 61) in which the mist discharging means and the mist dispersion means are connected; and
the unified unit has a discharge port (32a, 62a) of the mist discharging means and the plurality of transmitters disposed on the board surface of the board, respectively.

Moreover, the present invention is, according to the description in claim 4, the indoor decontamination device described in claim 3, characterized in that
the mist discharging dispersion device includes a plurality of the unified units; and
by directing a discharging dispersion direction of the decontamination mist by each of the unified units to different azimuths, respectively, the decontamination mist is discharged and dispersed in all the azimuths in the work room.

Moreover, the present invention is, according to the description in claim 5, the indoor decontamination device described in any one of claims 1 to 4, characterized in that
the connecting device includes an expansion/contraction mechanism which expands/contracts by the drive means; and
the mist discharging dispersion device is connected to an upper part of the decontamination device main-body via the connecting device and is moved in an up-down direction by an expansion/contraction of the connecting device.

Moreover, the present invention is, according to the description in claim 6, the indoor decontamination device described in any one of claims 1 to 4, characterized in that
the connecting device includes a rotation mechanism which rotates by the drive means; and
the mist discharging dispersion device is connected to an upper part of the decontamination device main-body via the connecting device and is rotated in a left-right direction by a rotation of the connecting device.

Moreover, the present invention is, according to the description in claim 7, the indoor decontamination device described in any one of claims 1 to 4, characterized in that
the connecting device includes a tilting mechanism which is tilted by the drive means; and
the mist discharging dispersion device is connected to an upper part of the decontamination device main-body via the connecting device and is tilted in an elevation-angle direction or a depression-angle direction by a tilting of the connecting device.

Moreover, the present invention is, according to the description in claim 8, the indoor decontamination device described in any one of claims 1 to 7, having:
at least any one of a temperature sensor (12, 42), a humidity sensor (12, 42), a gas concentration sensor (13, 43), and a condensation degree sensor (14, 44) and a control means, characterized in that
a decontamination operation is controlled based on a detection result of the sensor.

Moreover, the present invention is, according to the description in claim 9, the indoor decontamination device described in any one of claims 1 to 8, characterized in that
the indoor decontamination device includes a decomposition unit (16) which decomposes a mist and a gas of the decontamination chemical solution; and
the decomposition unit recovers the mist and the gas in the work room and performs a decomposition operation at an aeration after a decontamination operation.

Moreover, the present invention is, according to the description in claim 10, the indoor decontamination device described in any one of claims 1 to 9, including:
a floor-surface moving means (11) which travels/moves on a floor surface by the drive means, characterized by
traveling/moving by a manual manipulation or an automatic traveling inside the work room.

Moreover, the present invention is, according to the description in claim 11, the indoor decontamination device described in any one of claims 1 to 9, including:
a space moving means (47) which floats and moves in a space by the drive means, characterized by
performing a spade movement by a manual manipulation or an automatic flight in an internal space of the work room.

Moreover, the present invention is, according to the description in claim 12, the indoor decontamination device described in claim 10 or 11, including:
the floor-space moving means or the space moving means includes a three-dimensional distance measurement sensor, characterized by
performing an automatic traveling or an automatic flight while avoiding an obstacle inside the work room.

It is to be noted that signs in parentheses of each of the aforementioned means indicate a correspondence relationship with a specific means described in each of embodiments which will be described later.

### ADVANTAGEOUS EFFECTS OF INVENTION

According to the aforementioned configurations, the indoor decontamination device according to the present invention has a decontamination device main-body and a mist discharging dispersion device connected to the decontamination device main-body directly or via a connecting device. The decontamination device main-body includes a drive means and a liquid supply means which supplies a decontamination chemical solution to the mist discharging dispersion device. The mist discharging dispersion device includes a mist discharging means and a mist dispersion means.

The mist discharging means converts the decontamination chemical solution to the decontamination mist and supplies it to an inside of the work room. The mist dispersing means includes a board on which a plurality of transmitters are disposed and causes an acoustic flow by an ultrasonic wave to be generated in a vertical direction from a board surface of the board by subjecting the plurality of transmitters to a ultrasonic vibration. Thus, by causing a pressing force by an acoustic radiation pressure to act on the decontamination mist supplied to the inside of the work room, the decontamination mist is dispersed in the work room.

By configuring as above, an indoor decontamination device which can realize a perfect decontamination effect by employing a fine decontamination mist with a favorable decontamination efficiency and by supplying a proper amount of the decontamination mist to each corner of a decontamination target room and can improve an efficiency of a decontamination work by reducing a work time for an aeration or the like can be provided.

Moreover, according to the aforementioned configuration, the plurality of transmitters are disposed in the unified wave-transmission direction on the board surface of the board and operated in the same phase. As a result, ultrasonic waves in a front-surface direction of the plurality of transmitters reinforce each other, the ultrasonic waves in a lateral direction of the plurality of transmitters cancel each other, and an acoustic flow by the ultrasonic waves with a strong directivity in the vertical direction from the board surface of the board is generated. As a result, the aforementioned working effects can be exerted more specifically.

Moreover, according to the aforementioned configuration, the mist discharging dispersion device may include a unified unit in which a mist discharging means and a mist dispersion means are connected. The unified unit has a discharge port of the mist discharging means and a plurality of transmitters disposed on the board surface of the board respectively. As a result, the aforementioned working effects can be exerted more specifically.

Moreover, according to the aforementioned configuration, the mist discharging dispersion device may include a plurality of the unified units. It is to be noted that by directing a discharging dispersion direction of a decontamination mist by each of the unified units to azimuths different from each other, the decontamination mist can be discharged and dispersed in all the azimuths in a work room. As a result, the aforementioned working effects can be exerted more specifically and effectively.

Moreover, according to the aforementioned configuration, the connecting device may include an expansion/contraction mechanism that expands/contracts by a drive means. The mist discharging dispersion device is connected to an upper part of the decontamination device main-body via the connecting device and moves in an up-down direction by the expansion/contraction of the connecting device. By means of the movement of the mist discharging dispersion device in the up-down direction, the decontamination mist can be discharged and dispersed from an upper side to a lower side in the work room. As a result, the aforementioned working effects can be exerted more specifically and effectively.

Moreover, according to the aforementioned configuration, the connecting device may include a rotation mechanism which is rotated by the drive means. The mist discharging dispersion device is connected to the upper part of the decontamination device main-body via the connecting device and is rotated in a left-right direction by a rotation of the connecting device. By means of the rotation of the mist discharging dispersion device in the left-right direction, the decontamination mist can be discharged and dispersed to all the azimuths in the work room. As a result, the aforementioned working effects can be exerted more specifically and effectively.

Moreover, according to the aforementioned configuration, the connecting device may include a tilting mechanism which tilts by the drive means. The mist discharging dispersion device is connected to the upper part of the decontamination device main-body via the connecting device and tilts in an elevation-angle direction or a depression-angle direction by a tilting of the connecting device. By means of the tilting of the mist discharging dispersion device in the elevation-angle direction or the depression-angle direction, the decontamination mist can be discharged and dispersed from an upper side to a lower side in the work room. As a result, the aforementioned working effects can be exerted more specifically and effectively.

Moreover, according to the aforementioned configuration, the indoor decontamination device may have at least one sensor among a temperature sensor, a humidity sensor, a gas concentration sensor, and a condensation sensor and a control means. Thus, a decontamination operation can be controlled based on a detection result of each of the sensors. As a result, the aforementioned working effects can be exerted more specifically and effectively.

Moreover, according to the aforementioned configuration, the indoor decontamination device may include a decomposition unit which decomposes a mist and a gas of a decontamination chemical solution. This decomposition unit performs a decomposition operation by recovering the mist and the gas of the decontamination chemical solution in the work room at an aeration after the decontamination operation.

Moreover, according to the aforementioned configuration, the indoor decontamination device may include a floor-surface moving means which travels and moves on a floor surface by the drive means and travels and moves by a manual manipulation or an automatic traveling inside the work room. By means of a traveling and a movement of the indoor decontamination device, the decontamination mist can be discharged and dispersed to each corner in the work room. As a result, the aforementioned working effects can be exerted more specifically and effectively.

Moreover, according to the aforementioned configuration, the indoor decontamination device may include a space moving means which spatially moves by the drive means in an internal space of the work room by a manual manipulation or an automatic flight. By the spatial movement of the indoor decontamination device, the decontamination mist can be discharged and dispersed to each corner in the work room. As a result, the aforementioned working effects can be exerted more specifically and effectively.

Moreover, according to the aforementioned configuration, the floor-surface moving means or the space moving means may include a three-dimensional distance measurement sensor and perform an automatic traveling or an automatic flight while avoiding an obstacle inside the work room. As a result, the aforementioned working effects can be exerted more specifically and effectively.

### BRIEF DESCRIPTION OF DRAWINGS

FIG. 1 is a schematic diagram illustrating a state in which an indoor decontamination device which automatically travels, according to a first embodiment, is disposed in a clean room.
FIG. 2 is a perspective view of the indoor decontamination device which automatically travels, according to the first embodiment.
FIG. 3A is a front view and FIG. 3B is a side view of the indoor decontamination device which automatically travels, according to the first embodiment.
FIG. 4A is a front view of an indoor decontamination device which automatically moves in a space, according to a second embodiment.
FIG. 5B is a side view of the indoor decontamination device which automatically moves in a space, according to the second embodiment.

### DESCRIPTION OF EMBODIMENTS

In the present invention, a "mist" should be interpreted in a broad sense and is assumed to include a state of a liquid droplet of a decontamination agent which is refined and floats in an air, a state in which a gas and the liquid droplet of the decontamination agent are mixed, a state of the decontamination agent to repeat phase changes between a condensation and an evaporation of a gas and a liquid droplet and the like. Moreover, a grain diameter is also interpreted in a broad sense by including a mist, a fog, a liquid droplet and the like which are subclassified depending on a case.

Thus, in the mist according to the present invention, those called a mist (defined as 10 µm or less in some cases) or a fog (defined as 5 µm or less in some cases) and those having a grain diameter equal to or larger than them are assumed to be included in some cases. It is to be noted that, in the present invention, by means of an action of an ultrasonic vibration, even a mist, a fog, a liquid droplet and the like at 3 µm to 10 µm or the liquid droplet equal to or larger than that, it is considered that they are unified to a super-fine particle at 3 µm or less, and high-level decontamination effects are exerted.

Hereinafter, an indoor decontamination device according to the present invention will be described with reference to each of embodiments in detail. It is to be noted that the present invention is not limited only to each of the embodiments below.

### <First Embodiment>

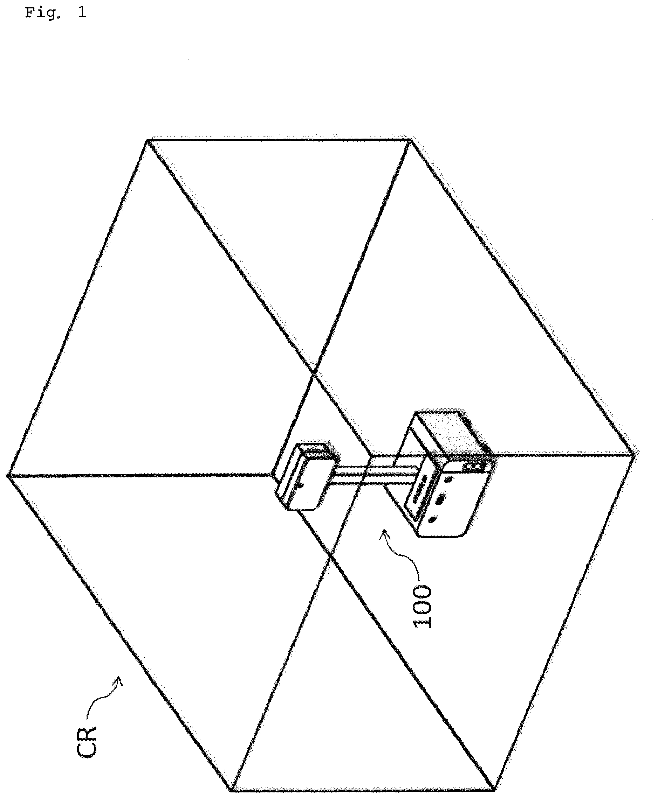

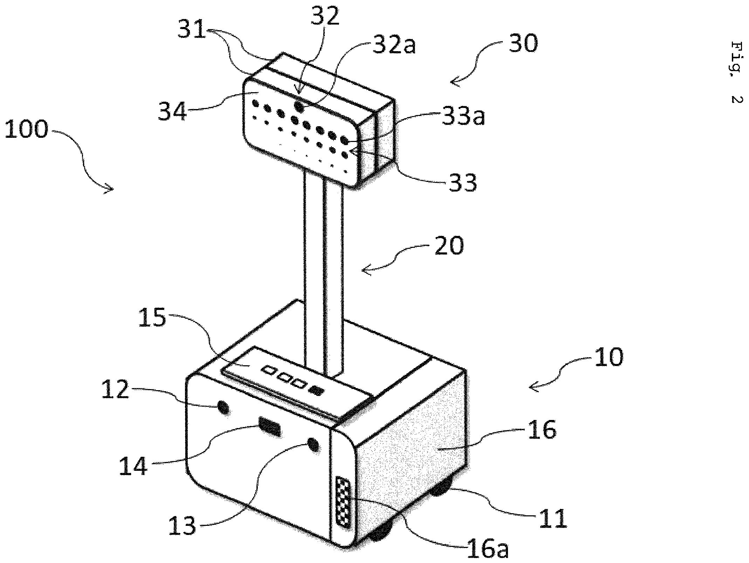

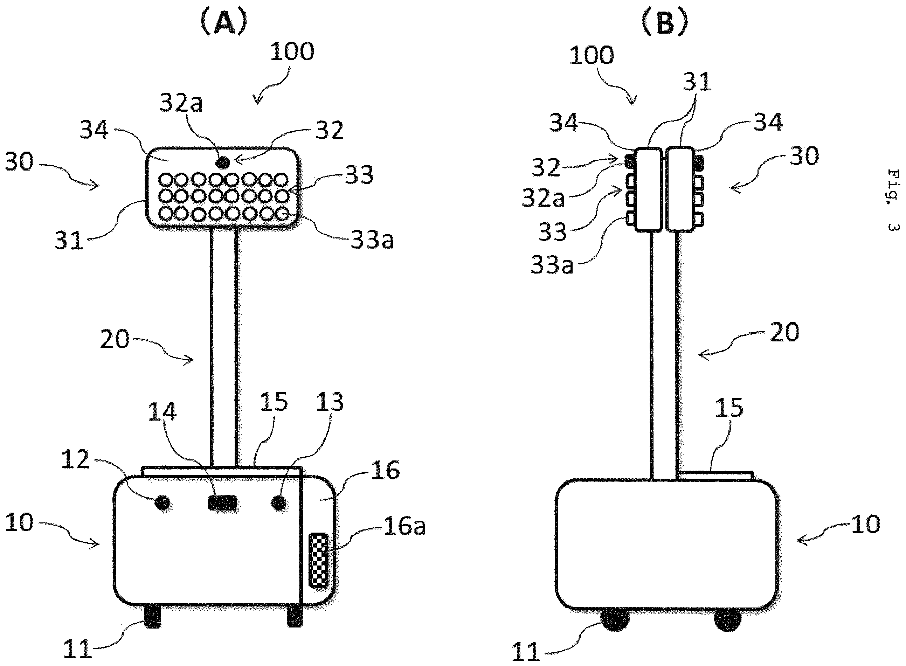

In this first embodiment, by using a clean room as an example of a work room to be decontaminated, an indoor decontamination device automatically traveling in a room will be explained. FIG. 1 is a schematic diagram illustrating a state in which an indoor decontamination device which automatically travels, according to a first embodiment, is disposed in a clean room. In FIG. 1, the indoor decontamination device 100 is disposed on a floor surface in a clean room CR. FIG. 2 is a perspective view of the indoor decontamination device 100 which automatically travels. FIG. 3A is a front view and FIG. 3B is a side view of the indoor decontamination device 100 which automatically travels.

In FIG. 2 and FIG. 3, in the indoor decontamination device 100, the mist discharging dispersion device 30 is connected to a decontamination device main-body 10 via a support 20. The decontamination device main-body 10 includes a drive device, a control device, a liquid supply tank storing a decontamination chemical solution and a liquid supply pump (none of them is shown) inside and supplies the decontamination chemical solution in the liquid supply tank to the mist discharging dispersion device 30 through a liquid-supply piping (not shown) disposed inside the support 20. It is to be noted that, in this first embodiment, a hydrogen peroxide solution (35 W/V%) was used as the decontamination chemical solution.

Moreover, on a bottom surface of the decontamination device main-body 10, a drive wheel 11 capable of changing a direction to drive by the drive device and the control device is provided and is controlled and automatically travels in the clean room. It is to be noted that a drive mechanism and a control method of an automatic traveling of the drive wheel 11 are not particularly limited, and it may travel in accordance with a program stored in advance in the control device by following a conventional automatic traveling-type device. Moreover, a power source of the drive device may be by an external power source or may be an internal power source such as a battery.

Moreover, the automatic traveling may be performed while uniformly decontaminating even a shaded part, while avoiding obstacles by detecting the obstacles in the clean room by a three-dimensional distance measurement sensor such as a TOF sensor (Time of Flight: image sensor which acquires a distance to a target and a shape of the target from a flight time of a light) accommodated inside the decontamination device main-body 10.

On a front surface wall of the decontamination device main-body 10, a temperature/humidity sensor 12, a gas concentration sensor 13, and a condensation degree sensor 14 are provided and detect a decontamination environment inside the clean room. Controls of these sensors are executed by the control device, and the control device controls a decontamination operation based on a detection result of each sensor. Here, the condensation degree sensor is a sensor proposed by the inventors before and can detect a condensed state of the hydrogen peroxide solution, which is a decontamination agent. Here, details of the condensation degree sensor will be omitted (see Japanese Patent Application Publication No. 3809176).

Moreover, on an upper surface wall of the decontamination device main-body 10, a control panel 15 is provided, and a worker can set decontamination conditions and the like before the decontamination operation. It is to be noted that, regarding the setting of the decontamination conditions and the like, a program created in advance may be incorporated in the control device. Moreover, a worker may wirelessly perform a manipulation from outside the clean room CR.

Moreover, on one of side surfaces of the decontamination device main-body 10, a decomposition unit 16 which decomposes a hydrogen peroxide is provided. The decomposition unit 16 includes a sucking/exhausting device and a catalyst device for the decomposition (not shown) inside the unit, a suction port 16a on a unit front surface, and an exhaust port (not shown) on a unit rear surface. This decomposition unit 16 decomposes a hydrogen peroxide solution mist which was discharged/dispersed into the clean room CR and the hydrogen peroxide solution gas which was evaporated from the hydrogen peroxide solution mist into the clean room CR at the aeration after the decontamination operation.

The support 20 is a connecting device which connects the decontamination device main-body 10 and the mist discharging dispersion device 30 to each other, and in this first embodiment, it is constituted by a double pipe including an expansion/contraction mechanism (not shown). The expansion/contraction mechanism of the support 20 vertically moves a mist discharging dispersion position of the mist discharging dispersion device 30 mounted on a distal end of the support 20. The expansion/contraction of the support 20 by the expansion/contraction mechanism is performed by the drive device and the control device provided inside the decontamination device main-body 10.

By the expansion/contraction of the support 20 of the indoor decontamination device 100 which automatically travels inside the clean room, the position of the mist discharging dispersion device 30 mounted on the distal end of the support 20 is vertically changed. As a result, the hydrogen peroxide solution mist can be uniformly discharged and dispersed to each corner (from an upper side to a lower side) inside the clean room. It is to be noted that the expansion/contraction mechanism of the support 20 and a control method are not particularly limited, and the expansion/contraction may be performed in accordance with a program stored in advance in the control device by following a conventional expanding/contracting device.

It is to be noted that the support 20 may include, in addition to or instead of the expansion/contraction mechanism, a rotation mechanism or a tilting mechanism. The rotation mechanism of the support 20 rotates a mist discharging/dispersion direction of the mist discharging dispersion device 30 mounted on the distal end of the support 20. A rotation of the mist discharging dispersion device 30 by the rotation mechanism is performed by the drive device and the control device provided inside the decontamination device main-body 10.

By the rotation of the mist discharging dispersion device 30 of the indoor decontamination device 100 which automatically travels in the clean room, the mist discharging dispersion direction is changed, and the hydrogen peroxide solution mist can be uniformly discharged and dispersed to each corner (to all azimuths) inside the clean room. It is to be noted that the rotation mechanism of the support 20 and a control method are not particularly limited, and the rotation may be performed in accordance with a program stored in advance in the control device by following the conventional expanding/contracting device.

The tilting mechanism of the support 20 tilts the mist discharging/dispersion direction of the mist discharging dispersion device 30 mounted on the distal end of the support 20 to an elevation-angle direction and a depression-angle direction. The tilting of the mist discharging dispersion device 30 by the tilting mechanism is performed by the drive device and the control device provided inside the decontamination device main-body 10.

By the tilting of the mist discharging dispersion device 30 in the indoor decontamination device 100 which automatically travels in the clean room in the elevation-angle direction and the depression-angle direction, the mist discharging direction is changed, the hydrogen peroxide solution mist can be uniformly discharged and dispersed to each corner (from an upper side to a lower side) inside the clean room. It is to be noted that the tilting mechanism of the support 20 and a control method are not particularly limited, and the tilting may be performed in accordance with a program stored in advance in the control device by following a conventional tilting device.

The mist discharging dispersion device 30 includes one or two or more units of the unified units 31 in which a mist discharging device 32 and a mist dispersion device 33 are connected. The unified unit has a discharge port 32a of the mist discharging device 32 and a plurality of transmitters 33a of the mist dispersion device 33 disposed on the board surface of the board 34, respectively.

It is to be noted that, in this first embodiment, the mist discharging dispersion device 30 has two units of the unified units 31 mounted on the distal end of the support 20 with rear surfaces thereof in contact with each other. The mist discharging device 32 of each of the unified units 31 converts a hydrogen peroxide solution supplied from the liquid supply tank provided inside the decontamination device main-body 10 to the hydrogen peroxide solution mist and supplies it into the clean room. It is to be noted that, in this first embodiment, an ultrasonic nebulizer was used as the mist discharging device 32.

It is to be noted that, in the present invention, a method of converting the hydrogen peroxide solution to the hydrogen peroxide solution mist is not limited to the ultrasonic nebulizer, and a mist generation mechanism and an output and the like are not particularly limited. For example, a two-fluid spray nozzle or the like may be used. When the two-fluid spray nozzle is used, the hydrogen peroxide solution is refined by a compressed air from a small-sized compressor provided in the decontamination device main-body 10 and discharged as the hydrogen peroxide solution mist into the clean room.

The mist dispersion device 33 in each of the unified units 31 subjects a plurality of the transmitters 33a (24 units in FIG. 3) disposed on the board surface of the board 34 to an ultrasonic vibration and causes an acoustic flow by an ultrasonic wave to be generated in a perpendicular direction from the board surface of the board 34. This acoustic flow causes a pressure by an acoustic radiation pressure to act on the hydrogen peroxide solution mist discharged in the perpendicular direction from the discharge port 32a of the mist discharging device 32 disposed on the board surface of the board 34 so that the hydrogen peroxide solution mist is further refined and dispersed in the clean room.

The plurality of transmitters 32a disposed on the board surface of the board 34 are disposed in a unified wave-transmission direction and are operated in the same phase. Therefore, ultrasonic waves vibrate such that the ultrasonic waves in a front-surface direction of each of the transmitters 32a reinforce each other and the ultrasonic waves in a lateral direction cancel each other. As a result, the mist dispersing device 32 can generate an acoustic flow by the ultrasonic waves with a strong directivity in the perpendicular direction from the board surface of the board 34.

It is to be noted that, in this first embodiment as described above, the mist discharging dispersion device 30 has two units of the unified units 31 mounted on the distal end of the support 20 with the rear surfaces thereof in contact with each other. As a result, the two units of the unified units 31 can discharge and disperse the hydrogen peroxide solution mist in directions different from each other by 180°. It is to be noted that the number of the unified units 31 is not limited to two, and three or more units may be disposed and directed to different directions. Moreover, even in a case where only one unit of the unified unit 31 is disposed, the hydrogen peroxide solution mist can be uniformly discharged and dispersed to each corner in the clean room by the expansion/contraction mechanism, the rotation mechanism, the tilting mechanism or a combination thereof.

Here, the plurality of transmitters 32a will be explained. In the first embodiment, an ultrasonic speaker was used as the transmitter 32a. On the board surface of the board 34, 24 units of the ultrasonic speakers are disposed with wave-transmission directions on their vibrating surfaces unified. It is to be noted that the number of ultrasonic speakers is not particularly limited.

In this first embodiment, an ultrasonic speaker with super directivity was used as the transmitter 32a. Specifically, a frequency-modulation type ultrasonic speaker (DC 12 V, 50 mA) which transmits ultrasonic waves with a frequency near 40 KHz was used. It is to be noted that a type, a size, a structure, an output and the like of the ultrasonic speaker are not particularly limited.

In this first embodiment, by unifying the wave transmission directions of the vibrating surfaces of the 24 units of ultrasonic speakers and by operating these transmitters in the same phase, the ultrasonic waves in the front surface direction of each of the ultrasonic speakers reinforce each other and the ultrasonic waves in the lateral direction of each of the ultrasonic speakers cancel each other. As a result, when the ultrasonic speakers disposed on the board surface of the board 34 ultrasonically vibrate, an acoustic flow with a strong directivity advancing in the air in a perpendicular direction from each of the vibrating surfaces is generated. It is to be noted that, by controlling a frequency and an output of the ultrasonic speaker by the control device (not shown), an efficient decontamination operation is made possible.

When the plurality of ultrasonic speakers supersonically vibrate, the acoustic flow with a strong directivity advancing in the perpendicular direction from the vibrating surface takes in the hydrogen peroxide solution mist discharged from the ultrasonic nebulizer, causes the pressure force by the acoustic radiation pressure to act on it, and moves it in the advancing direction of the acoustic flow. At this time, the hydrogen peroxide solution mist becomes a refined mist refined by an action of the ultrasonic vibration by the acoustic flow and is dispersed in the clean room.

At this time, the hydrogen peroxide solution mist which has become the refined mist is refined by an action of the ultrasonic vibration and has a small grain diameter and a large surface area and thus, it is considered that an evaporation efficiency of the mist is high, and an evaporation and a condensation are repeated. Moreover, the refined mist is a highly refined mist and forms a uniform and thin-layered condensed film on surfaces of various devices installed on an inner wall surface or an inside of the clean room. Therefore, as compared with a conventional decontamination operation, such a condensed film with a partial unevenness or thickness is not generated on the inner wall surface or the like of the clean room.

Moreover, by repeating a re-evaporation and the condensation of the condensed film formed uniformly and thin-layered on the inner wall surface of the clean room or the like, a hydrogen peroxide concentration in the decontamination mist can be raised, and an efficient decontamination with a small amount of the hydrogen peroxide is made possible. Moreover, since the efficient decontamination can be performed with a small amount of the hydrogen peroxide, an efficiency of an aeration after the decontamination is improved, and a time for the decontamination operation can be shortened. Further, since an amount of the hydrogen peroxide solution used for the decontamination of the inside of the clean room can be reduced, the indoor decontamination device 100 can be made small-sized and compact, and an automatic traveling can be made easy.

Thus, according to this first embodiment, an indoor decontamination device which can realize a perfect decontamination effect by employing the fine decontamination mist with a favorable decontamination efficiency and by supplying a proper amount of the decontamination mist to each corner of a decontamination target room and improve an efficiency of a decontamination work by shortening a work time for an aeration or the like can be provided.

### <Second Embodiment>

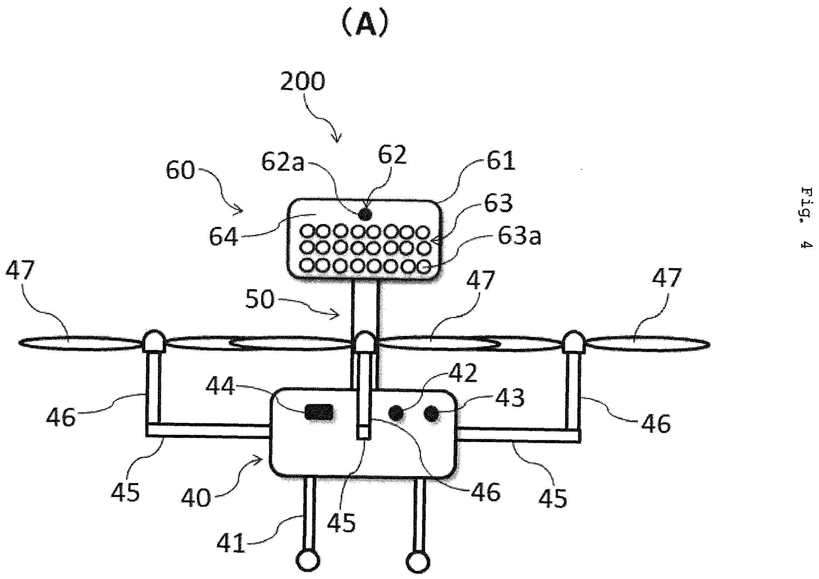

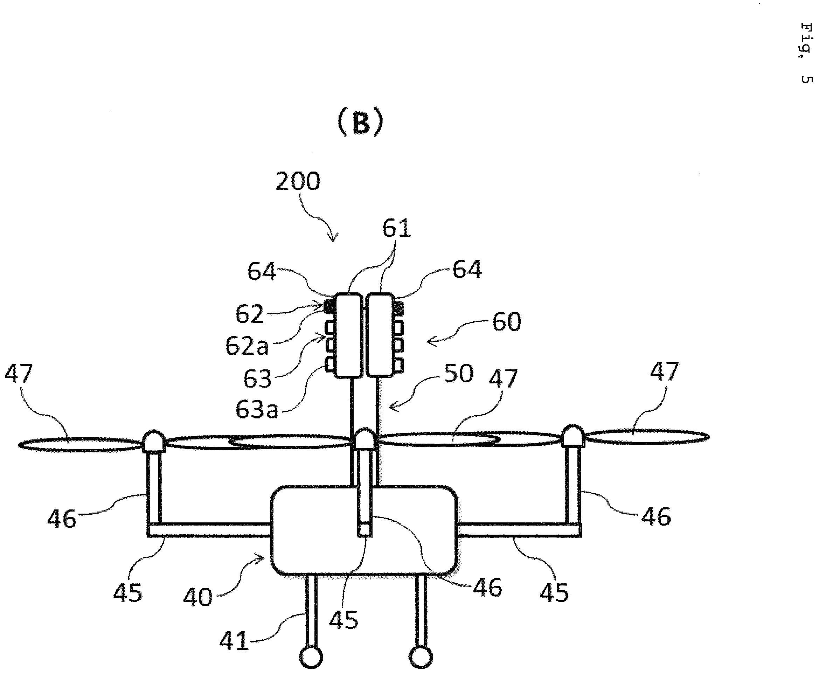

The aforementioned first embodiment is an indoor decontamination device which automatically travels in a clean room, while in this second embodiment, an indoor decontamination device which automatically moves in a space in a large clean room will be explained. FIG. 4A is a front view of the indoor decontamination device which automatically moves in the space according to the second embodiment. FIG. 5B is a side view of the indoor decontamination device which automatically moves in a space, according to the second embodiment.

In FIGs. 4 and FIGs. 5, an indoor decontamination device 200 has a mist discharging dispersion device 60 connected to a decontamination device main-body 40 via a support 50. Structures and functions of the decontamination device main-body 40, the support 50, and the mist discharging dispersion device 60 are similar to those of the aforementioned first embodiment and will be omitted here. It is to be noted that, in this second embodiment, a hydrogen peroxide solution (35 W/V%) was used as a decontamination chemical solution similarly to the aforementioned first embodiment.

On a bottom surface of the decontamination device main-body 40, a leg portion 41 which supports by a floor surface at taking-off/landing of the indoor decontamination device 200 is provided. Moreover, on a front surface wall of the decontamination device main-body 40, a temperature humidity sensor 42, a gas concentration sensor 43, and a condensation degree sensor 44 are provided and detect a decontamination environment in a clean room.

Moreover, on four side walls of the decontamination device main-body 40, horizontal support shafts 45 directed outward and vertical support shafts 46 directed upward from distal end parts of the horizontal support shafts 45 are provided, respectively, and propellers 47 from which the indoor decontamination device 200 obtains a lift are mounted on upper distal ends of the vertical support shafts 46. In this second embodiment, the indoor decontamination device 200 floats by the four units of propellers 47 mounted on the four side walls of the decontamination device main-body 40.

It is to be noted that, a control method of a drive mechanism of the propeller 47 and an automatic spatial movement is not particularly limited, and the spatial movement may be performed in accordance with a program stored in advance in the control device by following the conventional automatic flight device. Moreover, a power source of the drive device may be by an external power source or may be an internal power source such as a battery.

Moreover, the automatic spatial movement may be performed while uniformly decontaminating even a shaded part, while avoiding obstacles by detecting the obstacles in the clean room by a three-dimensional distance measurement sensor such as a TOF sensor accommodated inside the decontamination device main-body 40.

The mist discharging dispersion device 60 includes two units of unified units 61 in which a mist discharging device 62 and a mist dispersion device 63 are connected. The unified unit 61 has a discharge port 62a of the mist discharging device 62 and a plurality of transmitters 63a of the mist dispersion device 63 disposed on the board surface of a board 64 similarly to the aforementioned first embodiment.

It is to be noted that, in the present invention, as a method of converting a hydrogen peroxide solution to a hydrogen peroxide solution mist, an ultrasonic nebulizer was used similarly to the aforementioned first embodiment. Moreover, as the plurality of transmitters 63a (24 units in FIGs. 4) disposed on the board surface of the board 64 of the unified unit 61, an ultrasonic speaker with super directivity (DC 12V, 50 mA, transmitting ultrasonic waves with the frequency near 40 KHz) was used similarly to the aforementioned first embodiment.

In the configuration as above, the indoor decontamination device 200 automatically moves in a space inside the clean room. As a result, the indoor decontamination device 200 can discharge and disperse the decontamination mist to each corner in the work room. Moreover, since an amount of the hydrogen peroxide solution to be used can be reduced by refining of the hydrogen peroxide solution mist by an ultrasonic vibration, the indoor decontamination device 200 can be made small-sized and compact, and the automatic spatial movement can be made easy.

Thus, according to this second embodiment, an indoor decontamination device which can realize a perfect decontamination effect by employing a fine decontamination mist with a favorable decontamination efficiency and by supplying a proper amount of the decontamination mist to each corner of a decontamination target room and improve an efficiency of a decontamination work by shortening a work time for an aeration can be provided.

It is to be noted that, in working of the present invention, without limitation to each of the aforementioned embodiments, various variations can be cited as follows.
(1) In each of the aforementioned embodiments, the indoor decontamination device is caused to automatically travel or to automatically move in a space. However, this is not limiting, and in a relatively small clean room, the indoor decontamination device may be fixed and disposed at a center in the room as a device not using a traveling moving function or as a device not having the traveling moving function.
(2) In each of the aforementioned embodiments, the indoor decontamination device is caused to automatically travel or to automatically move in a space. However, this is not limiting, and it may be so configured that the indoor decontamination device is manually operated by a worker from outside the clean room.
(3) In each of the aforementioned embodiments, the decontamination device main-body and the mist discharging dispersion device are connected via a support as a connecting device a support. However, this is not limiting, and the decontamination device main-body and the mist discharging dispersion device may be directly connected.
(4) In each of the aforementioned embodiments, two units of the unified units are used as the mist discharging dispersion devices. However, this is not limiting, and one unit or three or more units of the unified units may be used.
(5) In each of the aforementioned embodiments, as the mist discharging dispersion device, the unified unit in which the mist discharging means and the mist dispersion means are connected is used. However, this is not limiting, and the mist discharging means and the mist dispersion means may be disposed separately.
(6) In each of the aforementioned embodiments, as the mist discharging device, an ultrasonic nebulizer was used. However, this is not limiting, and a two-fluid spray nozzle or other mist discharging means may be used.
(7) The indoor decontamination device according to the aforementioned first embodiment has a decomposition unit which decomposes a hydrogen peroxide solution mist and an evaporated hydrogen peroxide gas discharged into the clean room at an aeration after the decontamination operation. However, this is not limiting, and the decomposition unit may be installed at a different spot inside the clean room.

### REFERENCE SIGNS LIST

100, 200 Indoor decontamination device
10, 40 Decontamination device main-body
20, 50 Connecting device (support)
30, 60 Mist discharging dispersion device
11 Drive wheel
41 Leg portion
12, 42 Temperature/humidity sensor
13, 43 Gas concentration sensor
14, 44 Condensation degree sensor
15 Control panel
16 Decomposition unit
16a Suction port
31, 61 Unified unit
32, 62 Mist discharging device
32a, 62a Discharge port
33, 63 Mist dispersion device
33a, 63a Transmitter
34, 64 Board
45 Horizontal support shaft
46 Vertical support shaft
47 Propeller
CR Clean room

## Claims

1. An indoor decontamination device which is disposed inside a work room and decontaminates an inside of said entire room, having
a decontamination device main-body and a mist discharging dispersion device connected to said decontamination device main-body directly or via a connecting device, wherein
said decontamination device main-body includes a drive means and a supply means which supplies a decontamination chemical solution to said mist discharging dispersion device,
said mist discharging dispersion device includes a mist discharging means and a mist dispersion means,
said mist discharging means converts said decontamination chemical solution to a decontamination mist and supplies the decontamination mist into the inside of said work room, and
said mist dispersion means includes a board on which a plurality of transmitters are disposed, causes an acoustic wave by an ultrasonic wave to be generated in a vertical direction from a board surface of said board by subjecting said plurality of transmitters to an ultrasonic vibration, causes a pressing force by an acoustic radiation pressure to act on said decontamination mist supplied into said work room and disperses the decontamination mist in said work room.

2. The indoor decontamination device according to claim 1, **characterized in that**
said plurality of transmitters are disposed on a board surface of said board in a unified wave-transmission direction and are operated in a same phase, and
ultrasonic waves in a front-surface direction of said plurality of transmitters reinforce each other, said ultrasonic waves in a lateral direction of said plurality of transmitters cancel each other, and an acoustic flow by said ultrasonic waves with a strong directivity in a perpendicular direction is generated from said board surface of said board.

3. The indoor decontamination device according to claim 1 or 2, **characterized in that**
said mist discharging dispersion device includes a unified unit in which said mist discharging means and said mist dispersion means are connected, and
said unified unit has a discharge port of said mist discharging means and said plurality of transmitters disposed on a board surface of said board, respectively.

4. The indoor decontamination device according to claim 3, **characterized in that**
said mist discharging dispersion device includes a plurality of said unified units, and
by directing a discharging dispersion direction of said decontamination mist by each of said unified units to different azimuths, respectively, said decontamination mist is discharged and dispersed in all said azimuths in said work room.

5. The indoor decontamination device according to any one of claims 1 to 4, **characterized in that**
said connecting device includes an expansion/contraction mechanism which expands/contracts by said drive means, and
said mist discharging dispersion device is connected to an upper part of said decontamination device main-body via said connecting device and is moved in an up-down direction by an expansion/contraction of said connecting device.

6. The indoor decontamination device according to any one of claims 1 to 4, **characterized in that**
said connecting device includes a rotation mechanism which rotates by said drive means, and
said mist discharging dispersion device is connected to an upper part of said decontamination device main-body via said connecting device and is rotated in a left-right direction by a rotation of said connecting device.

7. The indoor decontamination device according to any one of claims 1 to 4, **characterized in that**
said connecting device includes a tilting mechanism which is tilted by said drive means, and
said mist discharging dispersion device is connected to an upper part of said decontamination device main-body via said connecting device and is tilted in an elevation-angle direction or a depression-angle direction by a tilting of said connecting device.

8. The indoor decontamination device according to any one of claims 1 to 7, having
at least any one of a temperature sensor, a humidity sensor, a gas concentration sensor, and a condensation degree sensor and a control means, wherein
a decontamination operation is controlled based on a detection result of said sensor.

9. The indoor decontamination device according to any one of claims 1 to 8, **characterized in that**
said indoor decontamination device includes a decomposition unit which decomposes a mist and a gas of said decontamination chemical solution, and
said decomposition unit recovers said mist and gas in said work room and performs a decomposition operation at an aeration after a decontamination operation.

10. The indoor decontamination device according to any one of claims 1 to 9, further comprising
a floor-surface moving means which travels and moves on a floor surface by said drive means, wherein
a traveling and moving is performed inside said work room by a manual manipulation or an automatic traveling.

11. The indoor decontamination device according to any one of claims 1 to 9, further comprising
a space moving means which floats and moves in a space by said drive means, **characterized by**
performing a space movement by a manual manipulation or an automatic flight in an internal space of said work room.

12. The indoor decontamination device according to claim 10 or 11, **characterized in that**
said floor-space moving means or space moving means includes a three-dimensional distance measurement sensor, and
an automatic traveling or an automatic flight is performed while avoiding an obstacle inside said work room.
